# EUROPEAN PATENT APPLICATION

(11) **EP 1 245 675 A1**
(43) Date of publication of application: **02.10.2002**
(21) Application number: 01400804.9
(22) Date of filing: 28.03.2001
(51) Int. Cl.: C12N 15/12, C12N 15/62, G01N 33/68, G01N 33/574, C07K 14/74, C07K 16/18

(54) **Cancer cell-specific HLA-F antigen and a diagnostic method of cancer by using thereof**

(71) Applicant: Egawa, Kohji, Tokyo (JP); Medinet Co., Ltd., Minato-ku, Tokyo (JP); Kimura, Yoshiji, Urayasu-shi, Chiba (JP)
(72) Inventor: Egawa, Kohji, Tokyo (JP)
(74) Representative: Michelet, Alain

(57) **Abstract**

This invention provides a method of detecting cancer cells in any organ and irrespective of causes of the tumors. In said method, a new antigenic substance that cancer cells commonly produce in a cancer cell-specific manner is first identified and, then, an antibody produced in response to this antigen is detected in body fluid of cancer patients. Specifically, this is achieved by detecting the anti-HLA-F antibody specific to the cancer cell-specific HLA-F antigen coded by the HLA-F gene.

SEQUENCE LISTING

<110> K. EGAWA et. al.

<120> Cancer Cell-Specific HLA-F Antigen and a Diagnostic Method of
Cancer by Using Thereof

<130>

<160> 6

<210> SEQ ID No:1
<211> 1089
<212> DNA
<213> human

<400> 1
atggcgcccc gaagcctcct cctgctgctc tcaggggccc tggccctgac cgatacttgg 60
gcgggctccc actccttgag gtatttcagc accgctgtgt cgcggcccgg ccgcggggag 120
ccccgctaca tcgccgtgga gtacgtagac gacacgcaat tcctgcggtt cgacagcgac 180
gccgcgattc cgaggatgga gccgcgggag ccgtgggtgg agcaagaggg gccgcagtat 240
tgggagtgga ccacagggta cgccaaggcc aacgcacaga ctgaccgagt ggccctgagg 300
aacctgctcc gccgctacaa ccagagcgag gctgggtctc acaccctcca gggaatgaat 360
ggctgcgaca tggggcccga cggacgcctc ctccgcgggt atcaccagca cgcgtacgac 420
ggcaaggatt acatctccct gaacgaggac ctgcgctcct ggaccgcggc ggacaccgtg 480
gctcagatca cccagcgctt ctatgaggca gaggaatatg cagaggagtt caggacctac 540
ctggagggcg agtgcctgga gttgctccgc agatacttgg agaatgggaa ggagacgcta 600
cagcgcgcag atcctccaaa ggcacacgtt gcccaccacc ccatctctga ccatgaggcc 660
accctgaggt gctgggccct gggcttctac cctgcggaga tcacgctgac ctggcagcgg 720

gatggggagg aacagaccca ggacacagag cttgtggaga ccaggcctgc aggggatgga 780
accttccaga agtgggccgc tgtggtggtg ccttctggag aggaacagag atacacatgc 840
catgtgcagc acgaggggct gccccagccc ctcatcctga gatgggagca gtctccccag 900
cccaccatcc ccatcgtggg catcgttgct ggccttgttg tccttggagc tgtggtcact 960
ggagctgtgg tcgctgctgt gatgtggagg aagaagagct cagatagaaa cagagggagc 1020
tactctcagg ctgcagtcac tgacagtgcc cagggctctg gggtgtctct cacagctaat 1080
aaagtgtga 1089

<210> SEQ ID No:2
<211> 822
<212> DNA
<213> human

<400> 2
ggctcccact ccttgaggta tttcagcacc gctgtgtcgc ggcccggccg cggggagccc 60
cgctacatcg ccgtggagta cgtagacgac acgcaattcc tgcggttcga cagcgacgcc 120
gcgattccga ggatggagcc gcgggagccg tgggtggagc aagaggggcc gcagtattgg 180
gagtggacca cagggtacgc caaggccaac gcacagactg accgagtggc cctgaggaac 240
ctgctccgcc gctacaacca gagcgaggct gggtctcaca ccctccaggg aatgaatggc 300
tgcgacatgg ggcccgacgg acgcctcctc cgcgggtatc accagcacgc gtacgacggc 360
aaggattaca tctccctgaa cgaggacctg cgctcctgga ccgcggcgga caccgtggct 420
cagatcaccc agcgcttcta tgaggcagag gaatatgcag aggagttcag gacctacctg 480
gagggcgagt gcctggagtt gctccgcaga tacttggaga atgggaagga gacgctacag 540
cgcgcagatc ctccaaaggc acacgttgcc caccacccca tctctgacca tgaggccacc 600
ctgaggtgct gggccctggg cttctaccct gcggagatca cgctgacctg gcagcgggat 660
ggggaggaac agacccagga cacagagctt gtggagacca ggcctgcagg ggatggaacc 720
ttccagaagt gggccgctgt ggtggtgcct tctggagagg aacagagata cacatgccat 780
gtgcagcacg aggggctgcc ccagcccctc atcctgagat gg 822

<210> SEQ ID No:3

<211> 645

<212> DNA

<213> human

<400> 3

atcgccgtgg agtacgtaga cgacacgcaa ttcctgcggt tcgacagcga cgccgcgatt 60

ccgaggatgg agccgcggga gccgtgggtg gagcaagagg ggccgcagta ttgggagtgg 120

accacagggt acgccaaggc caacgcacag actgaccgag tggccctgag gaacctgctc 180

cgccgctaca accagagcga ggctgggtct cacaccctcc agggaatgaa tggctgcgac 240

atggggcccg acggacgcct cctccgcggg tatcaccagc acgcgtacga cggcaaggat 300

tacatctccc tgaacgagga cctgcgctcc tggaccgcgg cggacaccgt ggctcagatc 360

acccagcgct tctatgaggc agaggaatat gcagaggagt tcaggaccta cctggagggc 420

gagtgcctgg agttgctccg cagatacttg gagaatggga aggagacgct acagcgcgca 480

gatcctccaa aggcacacgt tgcccaccac cccatctctg accatgaggc caccctgagg 540

tgctgggccc tgggcttcta ccctgcggag atcacgctga cctggcagcg ggatggggag 600

gaacagaccc aggacacaga gcttgtggag accaggcctg caggg 645

<210> SEQ ID No:4

<211> 362

<212> PRT

<213> human

<400> 4

Met Ala Pro Arg Ser Leu Leu Leu Leu Leu Ser Gly Ala Leu Ala Leu

1               5                   10                  15

Thr Asp Thr Trp Ala Gly Ser His Ser Leu Arg Tyr Phe Ser Thr Ala

            20                  25                  30

Val Ser Arg Pro Gly Arg Gly Glu Pro Arg Tyr Ile Ala Val Glu Tyr

        35                  40                  45
Val Asp Asp Thr Gln Phe Leu Arg Phe Asp Ser Asp Ala Ala Ile Pro
    50                  55                  60
Arg Met Glu Pro Arg Glu Pro Trp Val Glu Gln Glu Gly Pro Gln Tyr
65                  70                  75                  80
Trp Glu Trp Thr Thr Gly Tyr Ala Lys Ala Asn Ala Gln Thr Asp Arg
                85                  90                  95
Val Ala Leu Arg Asn Leu Leu Arg Arg Tyr Asn Gln Ser Glu Ala Gly
            100                 105                 110
Ser His Thr Leu Gln Gly Met Asn Gly Cys Asp Met Gly Pro Asp Gly
        115                 120                 125
Arg Leu Leu Arg Gly Tyr His Gln His Ala Tyr Asp Gly Lys Asp Tyr
    130                 135                 140
Ile Ser Leu Asn Glu Asp Leu Arg Ser Trp Thr Ala Ala Asp Thr Val
145                 150                 155                 160
Ala Gln Ile Thr Gln Arg Phe Tyr Glu Ala Glu Glu Tyr Ala Glu Glu
                165                 170                 175
Phe Arg Thr Tyr Leu Glu Gly Glu Cys Leu Glu Leu Leu Arg Arg Tyr
            180                 185                 190
Leu Glu Asn Gly Lys Glu Thr Leu Gln Arg Ala Asp Pro Pro Lys Ala
        195                 200                 205
His Val Ala His His Pro Ile Ser Asp His Glu Ala Thr Leu Arg Cys
    210                 215                 220
Trp Ala Leu Gly Phe Tyr Pro Ala Glu Ile Thr Leu Thr Trp Gln Arg
225                 230                 235                 240
Asp Gly Glu Glu Gln Thr Gln Asp Thr Glu Leu Val Glu Thr Arg Pro
                245                 250                 255
Ala Gly Asp Gly Thr Phe Gln Lys Trp Ala Ala Val Val Val Pro Ser
            260                 265                 270

Gly Glu Glu Gln Arg Tyr Thr Cys His Val Gln His Glu Gly Leu Pro
        275                 280                 285
Gln Pro Leu Ile Leu Arg Trp Glu Gln Ser Pro Gln Pro Thr Ile Pro
    290                 295                 300
Ile Val Gly Ile Val Ala Gly Leu Val Val Leu Gly Ala Val Val Thr
305                 310                 315                 320
Gly Ala Val Val Ala Ala Val Met Trp Arg Lys Lys Ser Ser Asp Arg
                325                 330                 335
Asn Arg Gly Ser Tyr Ser Gln Ala Ala Val Thr Asp Ser Ala Gln Gly
            340                 345                 350
Ser Gly Val Ser Leu Thr Ala Asn Lys Val
        355                 360

<210> SEQ ID No:5
<211> 274
<212> PRT
<213> human

<400> 5
Gly Ser His Ser Leu Arg Tyr Phe Ser Thr Ala Val Ser Arg Pro Gly
 1               5                  10                  15
Arg Gly Glu Pro Arg Tyr Ile Ala Val Glu Tyr Val Asp Asp Thr Gln
            20                  25                  30
Phe Leu Arg Phe Asp Ser Asp Ala Ala Ile Pro Arg Met Glu Pro Arg
        35                  40                  45
Glu Pro Trp Val Glu Gln Glu Gly Pro Gln Tyr Trp Glu Trp Thr Thr
    50                  55                  60
Gly Tyr Ala Lys Ala Asn Ala Gln Thr Asp Arg Val Ala Leu Arg Asn
65                  70                  75                  80

Leu Leu Arg Arg Tyr Asn Gln Ser Glu Ala Gly Ser His Thr Leu Gln
                85                  90                  95
Gly Met Asn Gly Cys Asp Met Gly Pro Asp Gly Arg Leu Leu Arg Gly
            100                 105                 110
Tyr His Gln His Ala Tyr Asp Gly Lys Asp Tyr Ile Ser Leu Asn Glu
        115                 120                 125
Asp Leu Arg Ser Trp Thr Ala Ala Asp Thr Val Ala Gln Ile Thr Gln
    130                 135                 140
Arg Phe Tyr Glu Ala Glu Glu Tyr Ala Glu Glu Phe Arg Thr Tyr Leu
145                 150                 155                 160
Glu Gly Glu Cys Leu Glu Leu Leu Arg Arg Tyr Leu Glu Asn Gly Lys
                165                 170                 175
Glu Thr Leu Gln Arg Ala Asp Pro Pro Lys Ala His Val Ala His His
            180                 185                 190
Pro Ile Ser Asp His Glu Ala Thr Leu Arg Cys Trp Ala Leu Gly Phe
        195                 200                 205
Tyr Pro Ala Glu Ile Thr Leu Thr Trp Gln Arg Asp Gly Glu Glu Gln
    210                 215                 220
Thr Gln Asp Thr Glu Leu Val Glu Thr Arg Pro Ala Gly Asp Gly Thr
225                 230                 235                 240
Phe Gln Lys Trp Ala Ala Val Val Val Pro Ser Gly Glu Glu Gln Arg
                245                 250                 255
Tyr Thr Cys His Val Gln His Glu Gly Leu Pro Gln Pro Leu Ile Leu
            260                 265                 270
Arg Trp

<210> SEQ ID No:6
<211> 215
<212> PRT

<213> human

<400> 6

Ile Ala Val Glu Tyr Val Asp Asp Thr Gln Phe Leu Arg Phe Asp Ser
1               5                   10                  15

Asp Ala Ala Ile Pro Arg Met Glu Pro Arg Glu Pro Trp Val Glu Gln
            20                  25                  30

Glu Gly Pro Gln Tyr Trp Glu Trp Thr Thr Gly Tyr Ala Lys Ala Asn
        35                  40                  45

Ala Gln Thr Asp Arg Val Ala Leu Arg Asn Leu Leu Arg Arg Tyr Asn
    50                  55                  60

Gln Ser Glu Ala Gly Ser His Thr Leu Gln Gly Met Asn Gly Cys Asp
65                  70                  75                  80

Met Gly Pro Asp Gly Arg Leu Leu Arg Gly Tyr His Gln His Ala Tyr
                85                  90                  95

Asp Gly Lys Asp Tyr Ile Ser Leu Asn Glu Asp Leu Arg Ser Trp Thr
            100                 105                 110

Ala Ala Asp Thr Val Ala Gln Ile Thr Gln Arg Phe Tyr Glu Ala Glu
        115                 120                 125

Glu Tyr Ala Glu Glu Phe Arg Thr Tyr Leu Glu Gly Glu Cys Leu Glu
    130                 135                 140

Leu Leu Arg Arg Tyr Leu Glu Asn Gly Lys Glu Thr Leu Gln Arg Ala
145                 150                 155                 160

Asp Pro Pro Lys Ala His Val Ala His His Pro Ile Ser Asp His Glu
                165                 170                 175

Ala Thr Leu Arg Cys Trp Ala Leu Gly Phe Tyr Pro Ala Glu Ile Thr
            180                 185                 190

Leu Thr Trp Gln Arg Asp Gly Glu Glu Gln Thr Gln Asp Thr Glu Leu
        195                 200                 205

Val Glu Thr Arg Pro Ala Gly

    210                 215

## Description

### Field of the Invention

This invention relates to a cancer cell-specific HLA-F antigen and a method of diagnosing cancer by using thereof; specifically, said novel HLA-F antigen is produced in cancer cells in a cancer cell specific manner, produced by gene recombination. Also, said method relates to detection of cancer cells by way of identifying an anti-HLA-F antibody produced as a result of immune reaction to such a cancer cell-specific HLA-F antigen.

### Background of the Art

For diagnosis of human cancer using biological specimen such as sera or the like, a method based on measurement of tumor markers has been devised. Available tumor markers include alpha fetoprotein (AFP) for liver cancer, carcinoembryonic antigen (CEA) for colon cancer, prostate specific antigen (PSA) for prostate cancer, and the like. As highly sensitive methods developed for measuring tumor markers, there are radioimmunoassay (RIA), enzyme immunoassay (EIA), fluoroimmunoassay (FIA), and the like, in which allogeneic monoclonal antibodies to tumor makers are employed.

Since the available tumor markers in the prior art described above are aimed at diagnosing cancer of specific organs and there are cancers in certain organs for which tumor markers are available, they cannot be applied to diagnosis of cancer in general. The fact that they are not exactly cancer-specific but are produced even in normal tissues to a certain extent makes it difficult to detect cancer in early stages, in which the amount of tumor markers is low. Furthermore, since these substances are not immunogenic in humans, immune reactions of the host to them cannot be utilized in diagnosing cancer.

On the other hand, in recent tumor immunology, identification of cancer antigenic peptides has attracted much attention. This trend was initiated with identification of the MAGE peptides of melanoma cells. Cancer antigenic peptides are brought to the cell surface by an antigen presentation mechanism. Some of the antigenic peptides are derived from abnormal proteins produced by mutations which cause malignant transformation of cells. Since such mutations are various, there are a variety of antigenic peptides specific for each cancer. For this reason, tumor antigen common to all cancers is yet to be discovered.

If there exists a substance which is exactly cancer-specific and not specific to organ, it may be used as a universal marker for cancer in general. It will be useful for the primary screening for the detection of cancer.

The object of this invention is to provide a method to detect cancer by identifying a novel antigenic substance that cancer cells produce commonly in a tumor-specific manner and by detecting an antibody produced in response to said antigen.

### Summary of the Invention

The inventors studied anti-tumor reactivity inherent to a living body using murine experimental cancer. As a result, they found that there exists an antigenicity common to various tumors and that host mouse responded by immune reactions including antibody production (T. Tanino, N. Seo, T. Okazaki, C. Nakanishi-Ito, M. Sekimata and K. Egawa, Cancer Immunol. Immunother, Vol. 35, p. 230 (1992)).

They have further identified one of the common antigens as a product of the Q5 gene that belongs to murine nonclassical histocompatibility class I antigen genes. These antigen genes are located adjacent to and are highly homologous to the normal histocompatibility class I antigen genes. Their function have not been elucidated.
Expression of the Q5 antigen, the product of the Q5 gene, has been confirmed in all experimental murine tumor cells tested irrespective of mouse strains, organs from which tumors derived, or causes of cancer (N. Seo, T. Okazaki, C. Nakanishi-Ito, T. Tanino, Y. Matsudaira, T. Takahashi and K. Egawa, J. Exp. Med., Vol. 17, No. 5, p. 647(1992)).

It was shown that immunization with Q5 antigen resulted in immune resistance to murine cancers in general (K. Egawa and N. Seo, Cancer Immunol. Immunother, Vol. 41, p. 384 (1995)).

The aforementioned studies of academic relevance deals specifically with mice ; therefore, these findings are not applicable directly to human subjects. This motivated the present inventors to further pursue a common tumor antigen in humans. It is well-known that human nonclassical histocompatibility class I antigen genes, comprising HLA-E, -F, -G, -H genes and the like, are highly homologous not only to each other but to -the classical histocompatibility class I antigen genes. However, little is known about the expression properties of the genes, nor functions thereof. These were underlying difficulties encountered when one wished to know which human nonclassical histocompatibility class I antigen would be a common antigen specifically related to a certain phenomenon.

When, however, the inventors studied if transcription of human nonclassical histocompatibility class I antigen genes took place using various cultured cells of human cancer, they obtained a surprising result that mRNAs in the HLA-F gene were commonly detected in these tumor cells.

They have, therefore, hypothesized that HLA-F gene is expressed in various cancer cells and that the gene product is antigenic to humans. When search was made, an anti-HLA-F antibodies was detected in sera from cancer patients.

Based on these findings, the inventors disclosed that HLA-F antigen, a product of the human HLA-F gene, is a tumor antigenic substance that cancer cells produce commonly in a cancer-specific manner. They further found that detection of antibodies to the cancer cell-specific HLA-F antigen in body fluid of human subjects could be interpreted as a proof for the presence of cancer. This completes the present invention.

The present invention, therefore, provides a cancer cell-specific HLA-F antigen which comprises at least the amino acid sequence described in SEQ ID No. 6 or 5 in the Sequence Listing. The antigen or the DNA that codes for it in the present invention refers to those, by containing certain amino acid or nucleotide sequences, that can be used for detecting anti-HLA-F antibodies which are present in body fluid of cancer patient. The present invention is claimed to all such antigens irrespective of names and origins, as long as they contain said sequences.

Furthermore, the invention provides a diagnostic method of cancer wherein said method detects an anti-HLA-F antibody in body fluid.

Yet another, the invention provides a detector of cancer wherein said detector comprises at least an introducer through which body fluid of a subject is introduced and an immunoreactor containing cancer cell-specific HLA-F antigen as its entirety or part of it.

The present invention is the first discovery of the existence of a human common cancer antigen, which is a breakthrough for the previous long-held common knowledge among cancer researchers that there would be no antigen which are shared by cancer cells in common and which is truly cancer-specific.

### Detailed Description of the Invention

### (1) Cancer Cell-Specific HLA-F Antigen

A cancer cell-specific HLA-F antigen, as the primary substance of the present invention, may be any antigen, as long as it binds as an antigen to an anti-HLA-F antibody in body fluid; specifically, said antigen comprises a part of the amino acid sequence described in SEQ ID No. 4 in the Sequence Listing. To be more specific, it contains at least an amino acid sequence in SEQ ID No. 5 and, preferably, at least an amino acid sequence in SEQ ID No. 6. Or, as long as it binds as an antigen to an anti-HLA-F antibody, it may be a part or an entirety of the amino acid sequence in SEQ ID No. 5 in the Sequence Listing or a part or an entirety of the amino acid sequence in SEQ ID No. 6. Amino acid sequences may be partially replaced, deleted, or added of a few amino acids by polymorphism in the species or by mutation without causing changes in their essential characteristics and; therefore, the claim of the present invention extends over such sequences, as long as they exert no fundamental modifications to the nature of the invention. The present inventors have identified that α₁, α₂, and α₃ extracellular domains of the α chain of the HLA-F antigen and, more specifically, the amino acid sequence in SEQ ID No. 6, are responsible for antigenicity of the cancer cell-specific HLA-F.

The cancer cell-specific HLA-F antigen is a product of the human HLA-F gene or a recombinant gene product using HLA-F cDNA which is synthesized from a mRNA separated from human cells. The nucleotide sequence of the HLA-F gene is described in SEQ ID No. 1 in the Sequence Listing.

Hence, the second substance of the present invention is a DNA that codes for the cancer cell-specific HLA-F antigen ; specifically, it comprises at least the DNA sequence described in SEQ ID No. 3 as its entirety or part of it, or at least the DNA sequence described in SEQ ID No. 2 as its entirety or part of it. Or, as long as it codes for the cancer cell-specific HLA-F antigen, the DNA may be of compressed or overlapped form of the DNAs comprising the sequences in SEQ ID No. 1, 2, and 3. Amino acid sequences of the cancer cell-specific HLA-F antigen may be partially replaced, deleted, or added of a few amino acids by polymorphism in the species or by mutation without causing changes in their essential characteristics; therefore, the claim of the present invention extends over DNAs coding for such amino acid sequences, as long as they exert no fundamental modifications to the nature of the invention.

Expression of the HLA-F gene can be confirmed using a method published by, e.g., J.M. Houlihan et al. (J. Immunology, Vol. 149, p. 668 (1992)) to detect HLA-F mRNA. The present invention is to detect cancer by way of identifying an HLA-F antibody induced as a result of immune reaction to the HLA-F antigen which is the product of the gene.

A cancer cell-specific HLA-F antigen may be purified directly from mass culture of tumor cells utilizing affinity of the antigenic peptide to anti-HLA-F antibody according to published methods, or purified from the products of recombinant gene or recombinant cDNA. For production of HLA-F, transformant may be prepared by introducing HLA-F gene or HLA-F cDNA, or more advantageously, by introducing recombinat DNA ,the products of which are fusion proteins containing HLA-F and vector derived amino acid sequences. In such cases, a procedure is designed to employ a specific protease in which the vector derived amino acid may be removed through cleavage at a recognition site of the enzyme.

In what follows, a method of preparing an HLA-F antigen is described in which expression of recombinant HLA-F cDNA is employed.

### (a) Synthesis of cDNA

Human cancer cells such as human myeloid leukemia cell HL-60 or U-973 are cultured and mRNA is prepared from them. cDNA to the mRNA is synthesized with the aid of reverse transcriptase. (See Cancer, Vol. 28, pp. 1300-1310 (1968) for more detailed information on the HL-60, and J. Exp. Med., Vol. 143, pp. 1528-1533 (1976) for U937. The description of the present invention refers to the literature cited to provide the relevant information.)

### (b) Preparation of HLA-F cDNA

The cDNA thus obtained is employed as the template DNA from which the HLA-F cDNA is amplified by way of the PCR (polymerase chain reaction) using HLA-F specific deoxyoligonucleotides as the primers. (See J. Immunology, Vol. 149, pp. 668-676 (1992) for further detail. The description of the present invention refers to the literature cited to provide the relevant information.)

The present inventors have revealed -that a part of the extracellular domains of the α chain of the HLA-F antigen is responsible for the antigenicity of the cancer cell-specific HLA-F antigen. Hence, the region of the cDNA coding for the extracellular domains of the α chain of the HLA-F antigen should preferably be amplified. Specifically, a primer designed to amplify an HLA-F cDNA fragment that contains at least No. 64 - No. 885 (SEQ ID No. 2) or, better yet, an HLA-F cDNA fragment that contains at least No. 130 - No. 774 (SEQ ID No. 3) should be used for amplification. Nucleotide sequence of the amplified cDNA is analyzed and compared the nucleotide sequence of the HLA-F gene for confirmation of that the amplified DNA is identical to the HLA-F cDNA.

### (c) Expression of HLA-F protein

Next, the amplified HLA-F cDNA is cloned into an expression vector such as pQE31. It is then used to transform *E. coli* and the like. The transformed cells are cultured and overexpression of HLA-F protein is induced.

### (d) Preparation/Purification of Cancer Cell-Specific HLA-F Antigen

*E. Coli* overexpressing the HLA-F protein thus obtained is solubilized and the HLA-F protein is purified by affinity purification method and the like. The cancer cell-specific HLA-F antigen thus obtained is an HLA-F protein fragment that the HLA-F gene codes for.

A fusion protein containing HLA-F and an amino acid sequence coded by an expression vector may be produced by transformation of *E. Coli* with the fusion gene. In such a case, the vector-coded amino acid sequence can be removed by the procedure hereafter described. A nucleotide sequence coding for the recognition sequence of a sequence-specific protease is inserted between the vector sequence and HLA-F cDNA. The resulting protein is purified and then cleaved by the protease to remove the vector-coded amino acid sequence.

When Enterokinase (Ekase) is used as the sequence-specific protease, a nucleotide sequence 5'-GACGACGACGACAAA-3', or other nucleotide sequences, that codes for the Ekase recognition amino acid sequence Asp-Asp-Asp-Asp-Lys is inserted between the vector sequence and HLA-F cDNA.

When Factor Xa is used as the sequence-specific protease, a nucleotide sequence 5'-ATCGAGGGCAGA-3', or other sequences, that codes for the Xa recognition amino acid sequence Ile-Glu-Gly-Arg is inserted between the vector sequence and HLA-F cDNA.

The cancer cell-specific HLA-F antigen is purified by fractionating the material and detecting the antigenic peptides. There is no restriction of the of fractionation. The liquid chromatography method using HPLC, FPLC, and the like, or the electrophoresis or any other appropriate method may be employed.

### (2) Method of Detecting Anti HLA-F Antibody

There is no restriction of the method of detecting an anti-HLA-F antibody, as long as said method utilizes immune reaction to a cancer cell-specific HLA-F antigen. Specifically, a cancer cell-specific HLA-F antigen is used as its entirety or part of it, thereby detecting an anti HLA-F antibody contained in body fluid of a subject. For example, this may be achieved by employing either the sandwich ELISA technique or the competition method; in case of the competition method, an immune complex formed between the cancer cell-specific HLA-F antigen as its entirety or part of it and a specific antibody to it is used to detect an anti-HLA-F antibody in the specimen which compete the binding of the antibody in the immune complex. An immune complex is a substance formed by immune reaction between HLA-F antigen as its entirety or part of it and a component, for example an anti HLA-F antibody, which reacts to it.

As a detector, any equipment may be employed, as long as it is composed of an introductory part, through which body fluid of a subject is introduced, and a part in which immune reaction to the cancer cell-specific HLA-F antigen as its entirety or part of it takes place. An exemplary immunoreactor may be materialized through solidication of a cancer cell-specific HLA-F antigen to a carrier. Detection of an anti-HLA-F antibody at the immunoreactor part may accomplished by invoking radio-immuno-assay (RIA), Western blotting, enzyme immuno-assay (EIA), fluorescent immuno-assay (FIA), and the like.

As a carrier on which a cancer cell-specific HLA-F antigen is solidified, any known material, such as nitrocellulose, polyvinylidenedifluoride (PVDF), a resin sheet or plate, latex or magnetic beads, and the like, may be successfully employed. Solidification of a cancer cell-specific HLA-F antigen may be accomplished through one of the following procedures: (a) a cancer cell-specific HLA-F antigen treated by SDS-polyacrylamide gel electrophoresis (SDS-PAGE) is blotted on a PVDF membrane, (b) a cancer cell-specific HLA-F antigen is bound noncovalently onto a resin plate surface, or (c) a cancer cell-specific HLA-F antigen is bound covalently to chemically activated magnetic beads, such as Dynabeads M450 Tosylactivated (manufactured by Dynal, Inc., Lake Success, NY). Alternatively, the detector described above and at least one of the reagents used for the detection may constitute a detection kit.

In what follows, the method of detecting an anti HLA-F antibody using Western blotting is described. After a cancer cell-specific HLA-F antigen is separated by way of SDS-polyacrylamide gel electrophoresis (SDS-PAGE), it is blotted on a PVDF membrane, such as Clearblot P membrane (manufactured by ATTO Corp ,Tokyo, Japan) and the like. From the resultant specimen, a filter for detection of an anti HLA-F antibody is prepared by blocking it by applying the PBS containing 1 % bovine serum albumin (BSA) and 5 % skim milk. The body fluid of a subject diluted 5 to 10 times with PBS containing 0.1 % of Tween 20 (referred to as T-PBS hereafter) is used as the source of primary antibody. The filter is reacted with the diluted body fluid for 90 minutes at 30 °C or 8 to 48 hours at 4 °C. After being washed carefully with T-PBS, it is further brought into reaction with T-PBS containing the secondary antibody comprising of the anti-human immunoglobulin goat antibody, anti-human immunoglobulin mouse antibody, anti-human immunoglobulin rabbit antibody, and the like, labeled with a marker such as biotin, enzyme, chemical color developer, or radioactive compound, and the like, for 90 minutes at 37 °C or 8 to 48 hours at 4 °C. After being washed again with T-PBS, the specimen is brought into reaction according to the marker bound to the secondary antibody, thereby detecting an anti-HLA-F antibody contained in the body fluid of the subject.

### (3) Diagnosis of Cancer

Upon detection of an anti-HLA-F antibody in the body fluid of a subject, the subject is diagnosed to have cancer in the body. If this is the case, it is preferable that the subject undergo further detailed tests by way of previously known methods for specification of the site of onset of cancer. For the testing, blood, plasma, serum, saliva, ascites, preural effusion, and the like, may be used, with particular preference given to serum.

### Examples

The present invention is hereinafter described in detail by referring to the Examples which by no means limit the scope of the present invention.

### EXAMPLE 1

### (1) Preparation of HLA-F cDNA from Cultured Cancer Cells

Following the procedure devised by J. M. Houlihan (J. Immunology, Vol. 149, pp. 668-676(1992)), RNA was extracted from U937 cells derived from human leukemia. From the RNA, mRNA having polyA structure was separated by using a ploydT column (Oligotex-dT30 manufactured by JSR Corp., Tokyo, Japan). This was used as the template to synthesize cDNA with the reverse transcriptase. Said cDNA was further used as the template in the amplification reaction using HLA-F α-chain specific primers (5'-ACATCGCCGTGGAGTACGTAGACG-3' and 3'-GAACACCTCTGGTCCGGACGTCCC-5'). The nucleotide sequence of the cDNA thus obtained was determined and then compared with the nucleotide sequence of the HLA-F gene, thereby identifying as a HLA-F cDNA fragment of 647 bp-long extending from exon 2 over exon 4. Said sequence comprises of the DNA sequence of the 5' end of SEQ ID. 3, to which ac is appended.

### (2) Preparation of cancer cell-specific HLA-F antigen

The HLA-F cDNA fragment obtained in (1) was ligated in the downstream region of the histidine tag DNA (His x6) in the expression vector pQE31. The base sequence 5'-GACGACGACGACAAA -3' coding for the Enterokinase recognition sequence (Ek) Asp-Asp-Asp-Asp-Lys was inserted between said histidine tag DNA and the HLA-F cDNA fragment. A recombinant plasmid was obtained by cloning. The E. *coli* JM109 line was transformed by said recombinant plasmid. Expression of the fusion protein of His x6 and the HLA-F fragment was induced in the resulting transformant by addition of isopropyl thiogalactopyranoside(IPTG) to the medium.

Said *E. coli* producing the fusion protein was disrupted by ultrasonication and insoluble material was separated. Said insoluble material was then made soluble by treating with urea and the fusion protein was purified from the lysate by Ni-chelate affinity chromatography. Analysis of the purified material on SDS-PAGE revealed that its molecular weight is 31 KD and the purity 95 %. Determination of N-terminal amino acid sequence proved that translation of the fusion protein takes place as expected.

Said purified fusion protein was treated with Enterokinase to obtain cancer cell-specific HLA-F antigen. SDS-PAGE analysis revealed, aside from the 31 KD molecular weight band not cleaved by the Enterokinase, the presence of 29 KD, 18 KD, and 13 KD bands. The amino acid sequences of the materials in these bands were determined. The results revealed that they were fragments of HLA-F gene products.

### (3) Detection of Anti HLA-F Antibody by Way of Western Blotting

The cancer cell-specific HLA-F antigen separated by SDS-PAGE was blotted on a Clearblot P membrane (manufactured by ATTO Corp., Tokyo, Japan), followed by blocking by applying the PBS containing 1 % bovine serum albumin (BSA) and 5 % skim milk to obtain a filter for detection of anti HLA-F antibody. This filter was used for detection of anti-HLA-F antibody.

Said filter for detection of anti-HLA-F antibody was submerged in 100 µl sera which are diluted 10 fold with T-PBS and kept for 90 minutes at 37°C for reaction. The sera were obtained from 52 subjects (32 cancer patients and 20 healthy subjects), and were used as the source of the primary antibody. After being washed carefully with T-PBS, it was further submerged in 1 ml of T-PBS containing 0.2 µl of the alkaline phosphatase-labeled anti-human IgG rabbit antibody (manufactured by Promega Corp., Madison, WI) used as the secondary antibody, and keep for 90 minutes at 37 °C or 8 to 48 hours at 4 °C. The resultant specimen is further washed by using T-PBS and brought into reaction with the alkaline phosphotase color development chemical ProtoBlot Western Blot AP System (manufactured by Promega Corp., Madison, WI).

### (4) Diagnosis of Cancer

When one of the 31KD, 29KD, 18KD or 13KD bands on the filter for detection of anti-HLA-F antibody showed positive color development, it was postulated that the result indicated the presence of anti-HLA-F antibody in the serum of the subject. This result, in turn, revealed the presence of cancer and was used as a basic for diagnosis of cancer.

Table 1 represents the results of color development on the anti-HLA-F antibody detection filter prepared according to the method described in (3). O shows positive color development in one of the 31 KD, 29 KD, 18 KD, or 13 KD molecular weight bands. When color development was observed in two or more bands, it was marked ⊕. If no color appears in any of the bands, it was marked ×.

### EXAMPLE 2

Instead of inserting the nucleotide sequence coding for the Enterokinase recognition sequence between the histidine tag DNA and the HLA-F cDNA fragment as in EXAMPLE 1 (2), the nucleotide sequence 5'-ATCGAGGGCAGA-3' coding for the Factor Xa recognition sequence Ile-Glu-Gly-Arg was inserted. The expressed fusion protein was processed for purification of the cancer cell-specific HLA-F antigen as in EXAMPLE 1, except it was treated with factor Xa (Protein Engineering Technology manufactured by Dynzyme ApS, Aarhus, Denmark). The cancer cell-specific HLA-F antigen thus obtained was analyzed with SDS-PAGE and molecular weight bands of 31 KD and 29 KD were distinctively observed.

Similar to EXAMPLE 1 above, detection of an anti-HLA-F antibody and cancer diagnosis are conducted on the 52 subjects including 32 cancer patients and 20 healthy donors. Table 1 represents the results of the color development on the anti-HLA-F antibody detection filter. When positive color development was observed in the 29 KD molecular weight band, it was marked O, while if no color appeared in the 29 KD band, it was marked ×.

### EXAMPLE 3

HLA-F cDNA is prepared from cultured cancer cells as in EXAMPLE 1, The HLA-F cDNA thus obtained was inserted in a fusion protein of glutathione-S-transferase (GST) expression vector. *E*. *coli* JM109 was transformed with the resulting recombinant plasmid and the GST-HLA-F fusion protein was obtained. The fusion protein thus obtained was solubilized in the presence of SDS and cleaved with thrombin to obtain a cancer cell-specific HLA-F antigen. SDS-PAGE of the thrombin digest showed 27.5 KD band of the GST and 25 KD band of the HLA-F fragment.

Similar to EXAMPLE 1, detection of anti-HLA-F antibody and cancer diagnosis were conducted on 20 subjects, including 13 cancer patients and 7 healthy subjects. It should be noted that, owing to a large amount of antibodies reactive with *E Coli* components present in sera of the subjects, color development somewhat lacks distinctiveness. Table 1 represents the results of the color development of the anti-HLA-F antibody detection filter. When distinct color was observed in the 25 KD band, it was marked with O, while if no color appeared in the 25 KD band, it was marked with ×.

**TABLE 1**

| Detection of Anti HLA-F Antibody | | | | | | |
|---|---|---|---|---|---|---|
| Subject | | | | | | |
| Cancer | Primary | Gender Age | | Result | | |
| Patient No. | Tumor site | | | EX.1 | EX.2 | EX.3 |
| 1 | liver | M | 53 | O | | O |
| 2 | stomach | M | 59 | O | O | O |
| 3 | liver | F | 62 | O | O | × |
| 4 | breast | F | 65 | ⊕ | O | O |
| 5 | lung | M | 46 | × | × | × |
| 6 | ovary | F | 63 | × | × | × |
| 7 | uterus | F | 44 | O | O | O |
| 8 | liver | M | 64 | O | O | O |
| 9 | ovary | F | 52 | × | × | - |
| 10 | liver, stomach | M | 70 | O | O | - |
| 11 | breast | F | 61 | × | × | × |
| 12 | liver | M | 77 | × | × | - |
| 13 | pancreas | F | 64 | ⊕ | O | - |
| 14 | histiocytoma | M | 58 | × | × | × |
| 15 | S colon | M | 56 | × | × | - |
| 16 | stomach | M | 48 | O | O | - |
| 17 | kidney | M | 63 | × | × | - |
| 18 | breast | F | 36 | × | × | - |
| 19 | ovary | F | 61 | O | O | - |
| 20 | lung | M | 62 | × | × | - |
| 21 | ovary | F | 52 | × | × | - |
| 22 | breast | F | 57 | × | × | - |
| 23 | ovary | F | 38 | × | × | - |
| 24 | lung | M | 58 | × | × | - |
| 25 | pancreas | M | 58 | O | O | - |
| 26 | pancreas | F | 58 | × | × | - |
| 27 | rectum | M | 56 | ⊕ | O | - |
| 28 | rectum | F | 65 | O | O | - |
| 29 | pancreas | M | 76 | × | × | - |
| 30 | lung | M | 50 | ⊕ | O | - |
| 31 | pancreas | F | 33 | × | × | - |
| 32 | tongue | M | 62 | O | O | - |
| 33 | S colon | M | 58 | - | - | × |
| 34 | breast | F | 52 | - | - | O |
| 35 | kidney | M | 64 | - | - | × |
| | | | | | | |

| Healthy Subject | | | | | | |
|---|---|---|---|---|---|---|
| 1 | | M | 62 | × | × | - |
| 2 | | M | 41 | × | × | - |
| 3 | | F | 60 | × | × | - |
| 4 | | F | 33 | × | × | - |
| 5 | | M | 36 | × | × | - |
| 6 | | M | 37 | × | × | - |
| 7 | | M | 42 | × | × | - |
| 8 | | F | 40 | × | × | - |
| 9 | | M | 39 | × | × | - |
| 10 | | M | 39 | × | × | - |
| 11 | | M | 26 | × | × | - |
| 12 | | M | 30 | × | × | - |
| 13 | | M | 31 | × | × | - |
| 14 | | M | 59 | × | × | - |
| 15 | | F | 23 | × | × | - |
| 16 | | F | 39 | × | × | - |
| 17 | | F | 34 | × | × | - |
| 18 | | F | 27 | × | × | ^{~} |
| 19 | | F | 23 | × | × | - |
| 20 | | F | 45 | × | × | - |
| 21 | | M | 40 | - | - | O |
| 22 | | M | 60 | - | - | × |
| 23 | | M | 28 | - | - | × |
| 24 | | M | 38 | - | - | × |
| 25 | | F | 25 | - | - | × |
| 26 | | M | 38 | - | - | × |
| 27 | | F | 34 | - | - | × |
| Note)Patient: Those who had been diagnosed cancer prior to the test Healthy: Those who had not been diagnosed cancer prior to the test -: Not tested | | | | | | |

As shown in Table 1, anti HLA-F antibody was detected in 16 out of 35 cancer patients, giving the detection rate of 45.7 %. With regard to those patients whose sera did not give positive color development on the filter, there was a possibility that the anti-HLA-F antibody in sera was neutralized by cancer cell-specific HLA-F antigen contained in the same sera, and hence, anti-HLA-F antibody was not detected. The 16 anti-HLA-F antibody positive patients had tumor of various initial sites. This result demonstrates the ability of the present invention to detect cancer inspective of the site of the onset. Therefore it is clear that cancer-cell of various organs commonly have cancer cell-specific HLA-F antigen of the present invention.

It may be noted that anti HLA-F antibodies was detected in one healthy subject (Healthy Subject 21). It was later revealed that said subject was diagnosed to have S colon cancer upon a detailed medical examination using the endoscope and the like after the present test. However, the values of CEA and CA19-9, which were widely used as markers of colon cancer, remained in the normal ranges ever at the time of diagnosis.

The cancer cell-specific HLA-F antigen aforementioned in the present invention is a new antigen substance that cancer cells produce commonly in a cancer cell-specific manner. By detecting an anti-HLA-F antibody that is produced in response to the cancer cell-specific HLA-F antigen in body fluid of a subject, it is possible to diagnose cancer irrespective of initial site or causes of cancer. Furthermore, it is thought to be highly effective in early detection of cancer that the presently available tumor markers fail.

## Claims

1. A cancer cell-specific HLA-F antigen wherein said antigen comprises at least a part of the amino acid sequence described in SEQ ID No. 6 in the Sequence Listing.

2. A cancer cell-specific HLA-F antigen wherein said antigen comprises at least a part of the amino acid sequence described in SEQ ID No. 5 in the Sequence Listing.

3. The cancer cell-specific HLA-F antigen according to either claim 1 or 2 wherein said antigen is obtained by expressing a DNA as its entirety or a part of it described in either SEQ ID No. 1, 2, or 3 in the Sequence Listing.

4. A DNA coding for a cancer cell-specific HLA-F antigen according to either claim 1, 2, or 3.

5. A method of preparing the cancer cell-specific HLA-F antigen according to either claim 1, 2, or 3 comprising the steps of:
producing a fusion protein using cells transformed by the DNA containing the entirety or a part of the nucleotide sequence described in either SEQ ID No. 1, 2, or 3 in the Sequence Listing; and
treating the fusion protein with a protease.

6. The method of preparing the cancer cell-specific HLA-F antigen according to claim 5 wherein said protease is Enterokinase.

7. The method of preparing the cancer cell-specific HLA-F antigen according to claim 5 wherein said protease is Factor Xa.

8. A method of preparing the cancer cell-specific HLA-F antigen according to either claim 1, 2, or 3 wherein said method further comprises a process of purification.

9. A diagnostic method of cancer comprising the step of detecting an anti HLA-F antibody of a subject by using a cancer cell-specific HLA-F antigen as its entirety or part of it.

10. A diagnostic method of cancer comprising the steps of:
competitively reacting a part of immunological pair which can be formed immune complex with a cancer cell-specific HLA-F antigen as its entirety or part of it, and an anti-HLA-F antibody in a body fluid of a subject; and
detecting an anti-HLA-F antibody in the body fluid of the individual.

11. The diagnostic method of cancer according to either claim 9 or 10 wherein the body fluid is a blood.

12. A detector of cancer comprising an introducer to which body fluid of an individual is introduced and an immunoreactor containing the cancer cell-specific HLA-F antigen as its entirety or part of it.

13. A detector kit of cancer comprising the detector according to claim 12 and at least one reagent for detection.
